# EUROPEAN PATENT SPECIFICATION

(11) **EP 3 402 408 B1**
(45) Date of publication and mention of the grant of the patent: **02.09.2020**
(21) Application number: 17701188.9
(22) Date of filing: 09.01.2017
(51) Int. Cl.: A61B 6/00, A61B 8/12, A61B 8/00, A61B 8/08

(54) **AUTOMATED PROBE STEERING TO CLINICAL VIEWS USING ANNOTATIONS IN A FUSED IMAGE GUIDANCE SYSTEM**
AUTOMATISIERTE SONDENLENKUNG AUF KLINISCHE ANSICHTEN MITTELS ANNOTATIONEN IN EINEM FUSIONSBILDFÜHRUNGSSYSTEM
DIRECTION DE SONDE AUTOMATISÉE POUR VUES CLINIQUES À L'AIDE D'ANNOTATIONS DANS UN SYSTÈME DE GUIDAGE D'IMAGES FUSIONNÉES

(30) Priority: 15.01.2016 US 201662279297 P
(43) Date of publication of application: 21.11.2018
(73) Proprietor: Koninklijke Philips N.V., 5656 AG Eindhoven (NL)
(72) Inventor: BRACKEN, John Allan, 5656 AE Eindhoven (NL); NOONAN, David Paul, 5656 AE Eindhoven (NL); POPOVIC, Aleksandra, 5656 AE Eindhoven (NL)
(74) Representative: Philips Intellectual Property & Standards
(86) International application number: PCT/IB2017/050075
(87) International publication number: WO 2017/122109

(56) References cited:
- WO-A1-2015/110882
- US-A1- 2013 259 341
- US-A1- 2015 016 704

## Description

### BACKGROUND:

### Technical Field

This disclosure relates to medical instruments and more particularly to systems and methods for automated steering and maintaining of clinical or standard views using image annotations.

### Description of the Related Art

During structural heart disease interventions, specific clinical views of an anatomy of interest are required for transesophageal echocardiography (TEE) imaging to carry out a specific task during the procedure. This task may include a positioning and deployment of a device within a target, a quantitative measurement of the target or an evaluation of the function of the target at a specific point in time. There are a number of different TEE views of targets needed in structural heart disease interventions that include, but are not limited to the 'en face' view of the mitral valve, mid-esophageal four-chamber view, long-axis view, transgastric view, tri-leaflet aortic valve view, x-plane view, etc. These views can help tremendously to enable the cardiologist and echocardiographer carry out a specific task within the patient, but they are often challenging to obtain and require continuous manual manipulation and adjustment of the TEE probe head within the patient by the echocardiographer to find and maintain the view. There is usually a large amount of discussion between the interventionalist and echocardiographer when determining a particular view for a specific task, since the interventionalist often provides feedback to the echocardiographer as to which view is needed. This can slow down the clinical workflow and make it more cumbersome.

Document WO 2015/110882 A1 describes robotic control of imaging devices.

### SUMMARY

In accordance with the present principles, an annotation device configured to generate annotations in images of a first imaging modality or a second imaging modality. A registration module is configured to fuse images of the first and second imaging modalities including the annotations. A robot guidance control system is configured to guide a robot in accordance with the annotations and a measured position of the robot to position and maintain an assigned view position in a fused image of the first and second imaging modalities.

Another imaging system includes a first imaging modality including an x-ray system and a second imaging modality including an ultrasound system with a transesophageal echocardiography (TEE) probe. An annotation device is configured to generate annotations in images of at least one of the first imaging modality or the second imaging modality. A registration module is configured to fuse images of the first and second imaging modalities including the annotations. A robot guidance control system is configured to guide a robot in accordance with the annotations and a measured position of the robot to position and maintain the probe to permit assigned views to be maintained. At least one display device has a screen to display a fused image of the first and second imaging modalities such that the fused image maintains an assigned view.

A method for maintaining an imaging perspective includes generating annotations in images of at least one of a first imaging modality or a second imaging modality; fusing images of the first and second imaging modalities including the annotations; and guiding a robot in accordance with the annotations and a measured position of the robot to position and maintain an assigned view position in a fused image of the first and second imaging modalities.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a system for directing or maintaining image view in multiple imaging modalities with annotated markings in accordance with one embodiment;
FIG. 2 is an image showing a mitral valve, aortic valve and left atrial appendage ostium and including automatically generated annotations for TEE views in accordance with one illustrative embodiment;
FIG. 3 is another annotated image including an elliptical annotation of an atrial septal defect in accordance with another illustrative embodiment;
FIG. 4 is another annotated image including a point marker annotation on an atrial septal defect in accordance with another illustrative embodiment;
FIG. 5 is a block/flow diagram showing a robotically controlled TEE positioning system in accordance with one embodiment; and
FIG. 6 is a flow diagram showing a method for maintaining an imaging perspective in accordance with illustrative embodiments.

### DETAILED DESCRIPTION OF EMBODIMENTS

In accordance with the present principles, systems and methods are provided that develop a more automated approach to determine transesophageal echocardiography (TEE) views or views in other procedures, using geometric feedback from digital annotations or markers placed on the anatomy in images. The images give feedback to a TEE probe robotic positioning system, which will assist in simplifying workflow for the echocardiographer and interventionalist during a procedure.

In structural heart disease interventions, TEE is an imaging modality (using ultrasound) that is often used to find viewing planes of the anatomy to facilitate execution of specific tasks during the intervention. It can be challenging for the echocardiographer to find a correct viewing plane, since substantial manual manipulation and adjustment (or correction) of the TEE probe position and orientation are needed. The present principles provide automated methods that enable the echocardiographer (or interventional cardiologist) to select a specific or pre-defined view for a particular target (e.g., 'en face' view of the mitral valve, mid-esophageal four-chamber view, long-axis view, transgastric view, tri-leaflet aortic valve view, etc.). These methods combine robotic manipulation of the TEE probe head position and orientation based on both the selected view of interest and feedback from annotations (or markers) placed on the target using an imaging system, e.g., a fused x-ray/TEE imaging system. These annotations can be placed or generated on a target in the x-ray and TEE modalities. Geometric descriptors of an annotation of a target (e.g., geometric coordinates, distances, orientation angles, etc.) can be used as feedback to the robotic control system and can orient and position the TEE probe head such that the desired view can be achieved.

Using a fused x-ray/TEE image guidance system, it is feasible to register x-ray and TEE images (ultrasound images) together into geometric correspondence with each other. With such a system (e.g., Philips® EchoNavigator™), it is possible to place or generate digital point markers, curves and ellipses as annotations on targets in the TEE or x-ray images. These annotations may include geometric information about the target. After an annotation has been manually placed or automatically generated on the target of interest using a fused x-ray/TEE image guidance system, and a desired view has been selected in such a system by the echocardiographer and/or the interventionalist, geometric information from the annotation(s) can be generated and delivered as continuous feedback to a robotic TEE probe positioning system. The robotic TEE probe positioning system adjusts probe position and orientation automatically to obtain the desired TEE view for the specific task in question. The present principles generate geometric data from annotations that have been placed on clinical targets in images on the fused x-ray/TEE system. These can be used as continuous feedback to automatically adjust TEE probe position after a desired TEE view has been selected.

It should be understood that the present invention will be described in terms of medical instruments; however, the teachings of the present invention are much broader and are applicable to any imaging instruments and imaging modalities. In some embodiments, the present principles are employed in tracking or analyzing complex biological or mechanical systems. In particular, the present principles are applicable to internal tracking procedures of biological systems and procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD

Reference in the specification to "one embodiment" or "an embodiment" of the present principles, as well as other variations thereof, means that a particular feature, structure, characteristic, and so forth described in connection with the embodiment is included in at least one embodiment of the present principles. Thus, the appearances of the phrase "in one embodiment" or "in an embodiment", as well any other variations, appearing in various places throughout the specification are not necessarily all referring to the same embodiment.

It is to be appreciated that the use of any of the following "/", "and/or", and "at least one of', for example, in the cases of "A/B", "A and/or B" and "at least one of A and B", is intended to encompass the selection of the first listed option (A) only, or the selection of the second listed option (B) only, or the selection of both options (A and B). As a further example, in the cases of "A, B, and/or C" and "at least one of A, B, and C", such phrasing is intended to encompass the selection of the first listed option (A) only, or the selection of the second listed option (B) only, or the selection of the third listed option (C) only, or the selection of the first and the second listed options (A and B) only, or the selection of the first and third listed options (A and C) only, or the selection of the second and third listed options (B and C) only, or the selection of all three options (A and B and C). This may be extended, as readily apparent by one of ordinary skill in this and related arts, for as many items listed.

It will also be understood that when an element such as a layer, region or material is referred to as being "on" or "over" another element, it can be directly on the other element or intervening elements may also be present. In contrast, when an element is referred to as being "directly on" or "directly over" another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

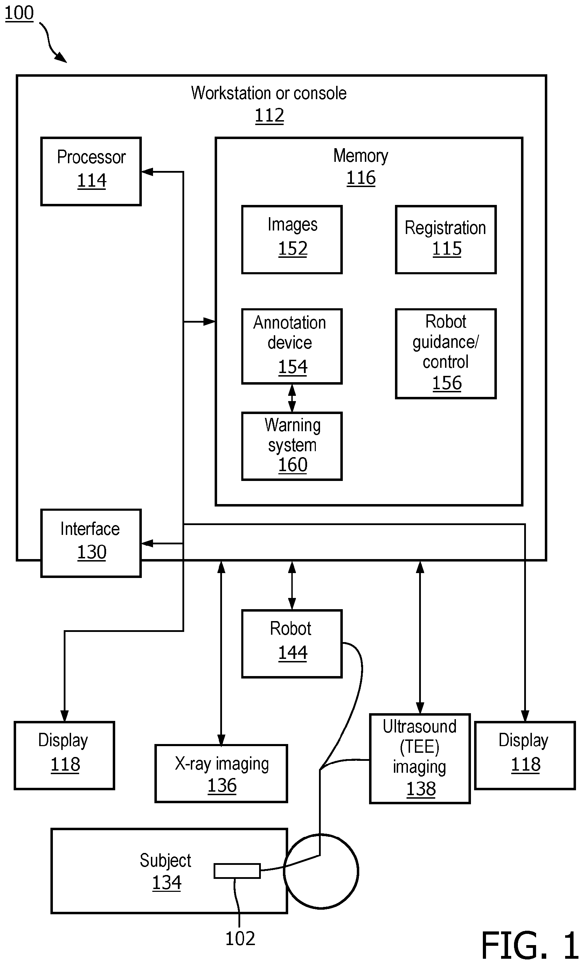

Referring now to the drawings in which like numerals represent the same or similar elements and initially to FIG. 1, a system 100 for directing or maintaining image view in multiple imaging modalities with annotated markings is illustratively shown in accordance with one embodiment. System 100 may include a workstation or console 112 from which a procedure is supervised and/or managed. Workstation 112 preferably includes one or more processors 114 and memory 116 for storing programs and applications. Memory 116 may store a registration module 115 configured to align coordinate systems from multiple imaging systems and/or devices. A medical device or instrument 102 may include a catheter, a guidewire, a probe, an endoscope, an electrode, a filter device, a balloon device, or other medical component, etc. The medical device 102 is passed into the subject through a natural orifice or through a port surgically installed through the subject to enter an internal lumen, such as the esophagus or the like.

The medical device 102 may be guided and controlled using a robot 144. The robot 144 is controlled by a robot guidance/control device 156. The guidance/control device 156 uses markings or other criteria set up in the system 100 by an annotation device 154 to guide the robot 144. The annotation device 154 includes markings or annotations input by a user or automatically generated using image processing to assist in achieving a plurality of different views. The views may be a predetermined/pre-defined set of views such that once the markings are in place the views can be achieved repeatably and maintained throughout a session or procedure.

In one embodiment, annotation device 154 is configured to receive feedback from the user or from another part of the system 100 and place markings within a preoperative image or real-time image from a first imaging system 136 (e.g., x-ray). The image from the first imaging system 136 is then registered to or fused with to an image from a second imaging system 138 (e.g., a TEE image) (or vice versa) using the registration module 115. Any suitable registration method may be employed.

Workstation 112 includes one or more displays 118 for viewing internal images of a subject (patient) or volume 134 and may include rendering images 152 as fused or as overlays of one other with the annotated markings. Displays 118 may also permit a user or users to interact with other users, or with the workstation 112 and its components and functions, or any other element within the system 100. This is further facilitated by an interface 130, which may include a keyboard, mouse, a joystick, a haptic device, speakers, microphone or any other peripheral or control to permit user feedback from and interaction with the workstation 112. The interface 130 may also be employed to permit the user or users to input annotations or markings to function as guideposts for the robot positioning and imaging.

In one particularly useful embodiment, system 100 is configured to perform transesophageal echocardiography (TEE) imaging for a procedure. In this embodiment, the imaging system 136 includes an x-ray system and the imaging system 138 includes a TEE imaging system. In a TEE procedure, an echocardiographer and an interventionalist may be present. A desired clinical TEE view is agreed upon by the echocardiographer and the interventionalist. The view may be a standard or commonly used view, (e.g., 'en face' view of the mitral or aortic valve, mid-esophageal four-chamber view, long-axis view, transgastric view, tri-leaflet aortic valve view, etc.). The selected view may be chosen on either the robot 144 (e.g., a robotic TEE probe positioning system) using TEE imaging 138 by the echocardiographer or on a fused x-ray/TEE image (registered TEE imaging 138 and x-ray imaging 136) by the interventionalist. In one embodiment, system 100 may be referred to as a fused x-ray/TEE image guidance system. Annotations relevant to the anatomy and view of interest may automatically be generated by the annotation device 154 on fused x-ray/TEE images 152 and displayed to both the interventionalist and echocardiographer on displays 118.

In one example, a curved annotation may be drawn (either automatically or manually) to outline commissures of the mitral valve. The 3D geometric information generated by this annotation could then be sent to the robot 144 to orient and position the TEE probe 102 for imaging 138 such that a clear 'en-face' view of the mitral valve can be displayed in 3D TEE images 152. These images 152 can then be used to align and position a mitral clip optimally between the mitral valve commissures before deploying the clip in a mitral clipping procedure.

As another example, an elliptical or curved annotation can be automatically or manually placed on the ostium of the left atrial appendage. The geometric information from this annotation can be sent as feedback to the robot 144, which can then automatically position the TEE probe 102 such that an optimal TEE view can be generated to help guide and deploy a closure device or plug in the left atrial appendage.

If the view is not standard or commonly used, an option to place annotations manually, or to automatically generate them on specific structures of interest, is provided to assist in inputting a desired view. Geometric descriptors of an annotation of a target (e.g., geometric coordinates, distances, orientation angles, etc.) can be used as feedback to the robotic guidance/control system 156 and can orient and position the TEE probe head (102) such that the desired view can be achieved. The necessary geometric data associated with the annotation(s) (e.g., 3D-coordinate positions, distances, normal vectors, angulation and curvature, centroids, area, major and minor axes, eccentricity, etc.) is sent as continuous feedback to the robot 144 (e.g., robotic TEE probe positioning system) to triangulate relative positions and orientations between the annotation(s) and the probe/instrument 102. This information can then be employed to position and deploy the probe 102 (e.g., TEE probe) appropriately in a continuous feedback loop to provide and maintain the desired TEE view.

In another embodiment, the repositioning of the probe 102 is not carried out automatically by the robot 144. Instead, the guidance control 156 may provide cues (e.g., sensory feedback, such as visual indicators on display 118, audio through interface 130, haptic feedback through interface, etc.) to enable an operator to actuate the necessary degrees of freedom of the robot 144 via the user interface 130, such as a joystick, to obtain an optimum visualization. In such an embodiment, the operator is 'in the loop' and the control software of the guidance control device 156 guides their actions using annotations as guide posts or references. For example, a sound (or visual signal) may indicate that the probe is getting closer or further from a desired position (annotation).

In another embodiment, a warning system 160 may be connected to the annotation device 154. The warning system 160 monitors any deviation of a current view from the intended (e.g., standard view). The warning system 160 may generate messages or provide color coding of annotations to indicate the amount and/or direction of the deviation. In one example, a color coded system may be generated on the fused x-ray/TEE image (or just the TEE image) to show how much the current TEE view deviates from the desired chosen view, (in terms of orientation, position, angle, etc.). This can assist whether the TEE probe position is manually or automatically adjusted. The warning system 160 could also be employed as a safety feature to tell the operator if a given view is not possible.

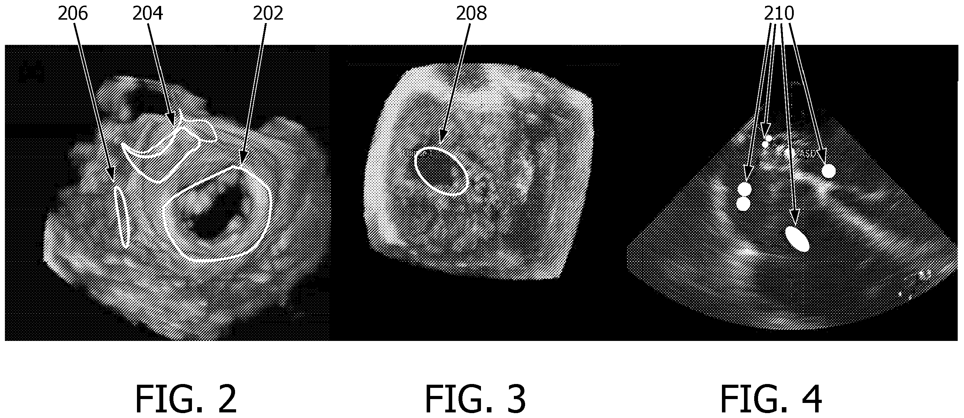

Referring to FIGS. 2-4 and initially to FIG. 2, an automatically generated annotation from a 3D-zoom TEE view of a mitral valve 202, aortic valve 204 and left atrial appendage ostium 206 are shown in accordance with one illustrative embodiment. The annotations are generated by the annotation device 154 or other software configured to make measurements based on features deciphered in the images. The annotations may include ellipses, points, lines, etc. between points of interest, etc. These annotations may be generated based on previous selections by the user to highlight different known features in a region. In FIG. 3, another annotated image includes an elliptical annotation 208 of an atrial septal defect. In FIG. 4, a point marker annotation 210 is shown on an atrial septal defect.

In FIG. 2, the examples of the automatically generated annotation of the mitral valve 202, aortic valve 204 and left atrial appendage ostium 206 are shown using a fused x-ray/TEE image. The system 100 is able to generate these annotations for 3D-zoom and full volume TEE views containing all three structures at once. With current fused x-ray/TEE imaging systems, the level of geometric detail in these annotations is currently limited to 3D position coordinates, but in accordance with the present principles, additional geometric data is employed for these annotations and can be generated and used as feedback to a robotic TEE positioning system to move the probe to a desired view. FIGS. 3 and 4 show less detailed annotations such as point markers 210 and ellipses 208 from which detailed geometric information can be generated to steer the TEE probe head to less conventional or non-standard clinical TEE views. An example of this could be steering to a color Doppler TEE view that shows a paravalvular leak in detail in an en face or long axis orientation, around a previously implanted prosthetic aortic valve.

In one embodiment, shapes and contours of the annotations (204-210) may be automatically and dynamically altered in the fused x-ray/TEE image as the anatomy of interest moves and changes. The corresponding geometric feedback to be delivered to the robot 144 (robotic TEE probe positioning system) may also be updated accordingly. For example, as the esophagus flexes so to do the shapes of the annotations (204-210). The robot 144 may be updated to adjust for these movements.

In another embodiment, interventional tools can be automatically annotated by the annotation device 154. For example, an interventional catheter can be detected and annotated in an x-ray image and that information can be used to automatically steer the TEE probe head 102 to less conventional or non-standard clinical TEE views showing the catheter. This device view can be saved alongside the standard clinical views. This is particularly useful if an interventional device is visible in the first imaging modality and not visible in the second imaging modality. In this case, the annotation can improve the device steering by depicting a virtual representation of the device in the second imaging modality using a geometric representation.

In another embodiment, an automatic generation of more detailed geometric data from less detailed geometric data may be provided. For example, if several points on an ellipse annotation (e.g., ellipse 208) are generated and known after placing the annotation, major and minor axes, area, eccentricity and the plane occupied may also be computed for the ellipse 208. From this plane, the normal to the ellipse 208 and the position of its center could be determined. This information may be important for the robot 144 (FIG. 1) to calculate how the probe 102 (FIG. 1) should be moved to visualize a target in the desired view.

Another example of this includes computing the centroid and normal of a 3D curve annotation, e.g., curve 206, which will provide information about its position and orientation relative to the TEE probe 102. Initially, the position of the center of a transducer of the probe head would be compared with the geometry of the annotations of interest for a particular view, but this could be extended to include additional details about the probe head transducer such as its area, dimensions and normal, to further assist with automatic probe positioning.

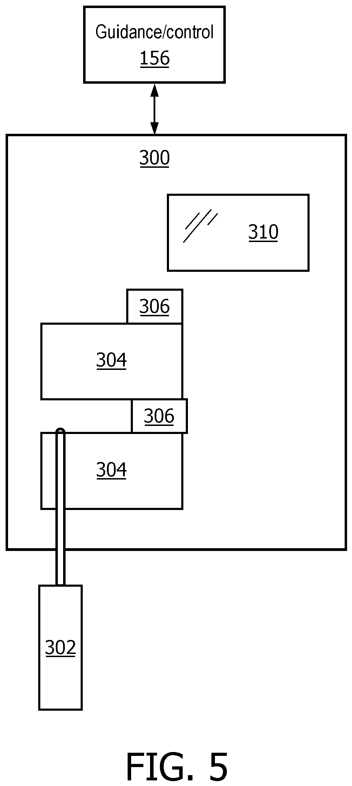

Referring to FIG. 5, a robotically controlled TEE positioning system 300 (e.g., robot 144, FIG. 1) is illustratively shown. The system 300 may be retrofitted with an existing TEE probe 302 and employed to remotely control a position and orientation of a distal tip of the probe 302. The system 300 includes an integrated motor or motors 304 and encoders 306 to provide position feedback to control software, e.g., robot guidance/control 156. The robot guidance/control 156 continuously monitors currents of the motor 304 and/or the encoders 306 to ensure the system 300 does not apply excessive force (or over extends) when in-vivo.

In one embodiment, the system 300 can accept geometric data from the annotations in the image views and combine the annotations with its own position and orientation data to compute instructions, e.g., how to actuate the probe 302 until a desired view is obtained. The geometric data can be sent to the system 300 from the workstation 112 (FIG. 1) in a continuous feedback loop such that the view can be maintained if the patient or probe is manually moved. In other words, the system 300 will continuously hold a view despite any movement. In one embodiment, the system 300 may include a display 310 such that a user (e.g., echocardiographer) can select the desired TEE view right on the system 300.

In one embodiment, the annotation device 154 (FIG. 1) may be included on the TEE imaging system 138. In this way, the annotation generation capability along with the position and orientation data generation for a control system of the robotic TEE probe positioning system 300 is directly on the TEE imaging system 138 (FIG. 1). This can be useful, in situations where fused x-ray/TEE imaging is not employed. In addition, the annotation capability may be extended to automatically (robotically) position less invasive transthoracic echocardiography (TTE) ultrasound imaging probes as well for specific views typically acquired with this modality.

The present principles improve image guidance and workflow for both the echocardiographer and interventionalist when using TEE and x-ray image guidance, e.g., in interventional cardiology and structural heart disease interventions. However, the present principles may be extended to other imaging modalities and procedures. The present principles may be employed as enhancements to systems such as, e.g., the Philips® EchoNavigator™ fused x-ray/TEE image guidance system, and CX50, iE33 and EPIQ ultrasound imaging systems.

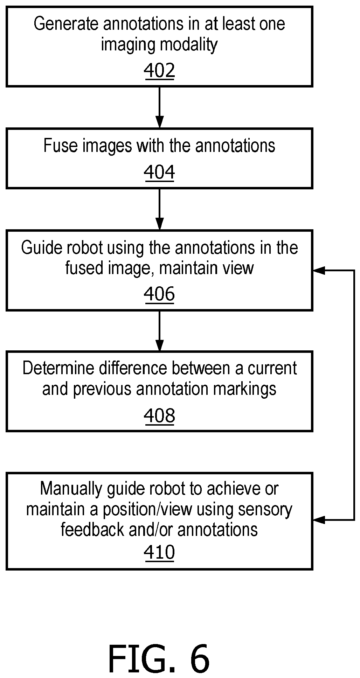

Referring to FIG. 6, a method for maintaining an imaging perspective is shown in accordance with the present principles. In block 402, annotations are generated in images of at least one imaging modality. The annotations may be generated automatically in accordance with physical features in a view or manually by adding annotations using a user interface.

In block 404, images of two or more imaging modalities are fused including the annotations. The imaging modalities may include an x-ray system and an ultrasound system where the probe may include a transesophageal echocardiography (TEE) probe.

In block 406, a robot is guided in accordance with the annotations and a measured position of the robot. This permits a position to be achieved and maintained for a probe (e.g., an ultrasound imaging probe or TEE probe) in an assigned view position in a fused image of the first and second imaging modalities. The assigned view may include a standard view or a view created by the user. The robot guidance may include tracking movement of the annotations to adjust the position of the probe. The annotations may be dynamically changed in accordance with patient or device movement, the robot tracking may continue using the updated annotations.

In block 408, differences between annotations may be monitored between a current view with annotations and a previous view. The changes may be indicated or employ a color coded system to show deviations. A warning system may be provided to alert a user of the deviations or to provide guidance to the user for manually achieving or maintaining an assigned view. In block 410, the robot may be manually guided using sensory feedback and/or the annotations as a reference to achieve and maintain a position.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for automated probe steering to clinical views using annotations in a fused image guidance system (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. An imaging system, comprising:
an annotation device (154) configured to generate annotations of anatomical targets (202, 204, 206, 208, 210) in images of at least one of a first imaging modality or a second imaging modality, each annotation comprising a geometric descriptor of the anatomical target, the geometric descriptor comprising geometric coordinates and an orientation angle for use in achieving a desired view of the anatomical target;
a registration module (115) configured to fuse images of the first and second imaging modalities including the annotations; and
a robot guidance control system (156) configured to guide a robot (144) in accordance with the annotations and a measured position of the robot to position and maintain an assigned view position and orientation in a fused image of the first and second imaging modalities.

2. The system as recited in claim 1, wherein the first imaging modality (136) includes an x-ray system and the second imaging modality (138) includes an ultrasound probe.

3. The system as recited in claim 2, wherein the ultrasound system includes a transesophageal echocardiography (TEE) system.

4. The system as recited in claim 3, wherein the assigned view includes one of 'en face' view, mid-esophageal four-chamber view, long-axis view, transgastric view, tri-leaflet aortic valve view and an x-plane view.

5. The system as recited in claim 1, wherein the annotation device (154) is configured to automatically generate annotations associated with physical features in a view.

6. The system as recited in claim 5, wherein automatically generated annotations may include one of features or interventional devices in the view.

7. The system as recited in claim 5, wherein the annotations (202) include geometric shapes, contours, measurements and marked positions.

8. The system as recited in claim 2, wherein the robot guidance control system (156) tracks movement of the annotations to adjust the position of the probe.

9. The system as recited in claim 1, further comprising an interface (130) for manually adding annotations through the annotation device.

10. The system as recited in claim 1, further comprising a warning system (160) coupled to the annotation device to monitor differences between annotations in a current view with annotations in a previous view.

11. The system as recited in claim 1, further comprising an interface (130) for manually guiding the robot using sensory feedback and the annotations as a reference.

12. An imaging system, comprising:
a first imaging modality (136) including an x-ray system and a second imaging modality (138) including an ultrasound system with a transesophageal echocardiography (TEE) probe;
an annotation device (154) configured to generate annotations of anatomical targets (202, 204, 206, 208, 210) in images of at least one of the first imaging modality or the second imaging modality, each annotation comprising a geometric descriptor of the anatomical target, the geometric descriptor comprising geometric coordinates and an orientation angle for use in achieving a desired view of the anatomical target;
a registration module (115) configured to fuse images of the first and second imaging modalities including the annotations;
a robot guidance control system (156) configured to guide a robot in accordance with the annotations and a measured position of the robot to position and maintain the probe to permit assigned view positions and orientations to be maintained; and
at least one display device having a screen to display a fused image of the first and second imaging modalities such that the fused image maintains an assigned view position and orientation.

13. The system as recited in claim 10, wherein the assigned view includes one of 'en face' view, mid-esophageal four-chamber view, long-axis view, transgastric view, tri-leaflet aortic valve view and an x-plane view.

14. The system as recited in claim 12, wherein the annotation device (154) is configured to automatically generate annotations associated with physical features in a view.

15. The system as recited in claim 12, wherein the annotations (202) include geometric shapes, contours, measurements and marked positions.

## Patentansprüche

1. Ein Bildgebungssystem, das Folgendes umfasst:
eine Anmerkungsvorrichtung (154), die Anmerkungen für anatomische Ziele (202, 204, 206, 208, 210) in Bildern mindestens eines ersten oder zweiten Bildgebungsverfahrens erstellt, wobei die einzelnen Anmerkungen einen geometrischen Deskriptor des anatomischen Ziels umfassen, der die geometrischen Koordinaten sowie den Lagewinkel für die gewünschte Ansicht des anatomischen Ziels enthält;
ein Registrierungsmodul (115) zum Zusammenführen er Bilder des ersten und zweiten Bildgebungsverfahrens sowie der Anmerkungen; und
ein Steuerungssystem für die Roboterführung (156), das einen Roboter (144) in Übereinstimmung mit den Anmerkungen und einer gemessenen Position des Roboters in Position bringt und die zugewiesene Bildposition und Ausrichtung für das zusammengeführte Bild eines ersten und zweiten Bildgebungsverfahrens beibehält.

2. Das System gemäß Anspruch 1, wobei das erste Bildgebungsverfahren (136) ein Röntgensystem und das zweite Bildgebungsverfahren (138) eine Ultraschallsonde umfasst.

3. Das System gemäß Anspruch 2, wobei das Ultraschallsystem ein zudem ein transösophageales Echokardiographie-System (TEE) umfasst.

4. Das System gemäß Anspruch 3, wobei die zugewiesene Ansicht eine der folgenden Ansichten umfasst: "En-Face", mittig ösophageal, Längsachse, transgastrisch, Dreifach-Segel-Aortenklappe und X-Ebene.

5. Das System gemäß Anspruch 1, wobei die Anmerkungsvorrichtung (154) automatisch die den physischen Merkmalen der Ansicht zugeordneten Anmerkungen erstellt.

6. Das System gemäß Anspruch 5, wobei die automatisch erstellten Anmerkungen die Merkmale oder Interventionsgeräte enthalten können.

7. Das System gemäß Anspruch 5, wobei die Anmerkungen (202) geometrische Formen, Konturen, Messungen und markierte Positionen umfassen.

8. Das System gemäß Anspruch 2,
wobei das Steuerungssystem für die Roboterführung (156) zum Anpassen der Position der Sonde die Bewegung der Anmerkungen nachverfolgt.

9. Das System gemäß Anspruch 1, wobei dieses zudem über eine Schnittstelle (130) zum manuellen Hinzufügen von Anmerkungen mit der Anmerkungsvorrichtung verfügt.

10. Das System gemäß Anspruch 1, wobei dieses zudem ein Warnsystem (160) umfasst, das mit der Anmerkungsvorrichtung verbunden ist, um die Unterschiede zwischen den Anmerkungen einer aktuellen und einer vorherigen Ansicht zu überwachen.

11. Das System gemäß Anspruch 1, wobei dieses zudem über eine Schnittstelle (130) zum manuellen Führen des Roboters anhand der sensorischen Rückmeldungen und der Anmerkungen.

12. Ein Bildgebungssystem, das Folgendes umfasst:
ein erstes Bildgebungsverfahren (136) mit einem Röntgensystem und ein zweites Bildgebungsverfahren (138) mit einem Ultraschallsystem mit einer transösophagealen Echokardiographie-Sonde (TEE) probe;
eine Anmerkungsvorrichtung (154), die Anmerkungen für anatomische Ziele (202, 204, 206, 208, 210) in Bildern mindestens des ersten oder zweiten Bildgebungsverfahrens erstellt, wobei die einzelnen Anmerkungen einen geometrischen Deskriptor des anatomischen Ziels umfassen, der die geometrischen Koordinaten sowie den Lagewinkel für die gewünschte Ansicht des anatomischen Ziels enthält;
ein Registrierungsmodul (115) zum Zusammenführen er Bilder des ersten und zweiten Bildgebungsverfahrens sowie der Anmerkungen;
ein Steuerungssystem für die Roboterführung (156), das einen Roboter in Übereinstimmung mit den Anmerkungen und einer gemessenen Position des Roboters in Position bringt und die Sonde in Position hält, um die zugewiesenen Bildpositionen und Ausrichtungen beizubehalten; und
mindestens ein Anzeigegerät mit einem Bildschirm, auf dem das zusammengeführte Bild des ersten und zweiten Bildgebungsverfahren so angezeigt wird, dass das zusammengeführte Bild die zugewiesene Anzeigeposition und Ausrichtung beibehält.

13. Das System gemäß Anspruch 10, wobei die zugewiesene Ansicht eine der folgenden Ansichten umfasst: "En-Face", mittig ösophageal, Längsachse, transgastrisch, Dreifach-Segel-Aortenklappe und X-Ebene.

14. Das System gemäß Anspruch 12, wobei die Anmerkungsvorrichtung (154) automatisch die den physischen Merkmalen der Ansicht zugeordneten Anmerkungen erstellt.

15. Das System gemäß Anspruch 12, wobei die Anmerkungen (202) geometrische Formen, Konturen, Messungen und markierte Positionen umfassen.

## Revendications

1. Système d'imagerie, comprenant :
un dispositif d'annotation (154) configuré pour générer des annotations des cibles anatomiques (202, 204, 206, 208, 210) dans des images d'une première modalité d'imagerie et/ou d'une seconde modalité d'imagerie, chaque annotation comprenant un descripteur géométrique de la cible anatomique, le descripteur géométrique comprenant des coordonnées géométriques et un angle d'orientation à utiliser pour obtenir une vue souhaitée de la cible anatomique ;
un module d'enregistrement (115) configuré pour fusionner des images des première et seconde modalités d'imagerie comprenant les annotations ; et
un système de commande du guidage robotique (156) configuré pour guider un robot (144) en fonction des annotations et d'une position mesurée du robot pour positionner et maintenir une position et une orientation de vue attribuées dans une image fusionnée des première et seconde modalités d'imagerie.

2. Système selon la revendication 1, dans lequel la première modalité d'imagerie (136) comprend un système à rayons X et la seconde modalité d'imagerie (138) comprend une sonde à ultrasons.

3. Système selon la revendication 2, dans lequel le système à ultrasons comprend un système d'échocardiographie transœsophagienne (TEE).

4. Système selon la revendication 3, dans lequel la vue attribuée comprend une vue « en face » ou une vue mi-œsophagienne 4-chambre ou une vue selon l'axe longitudinal ou une vue transgastrique ou une vue aortique 3-feuillets valvulaires ou une vue dans le plan x.

5. Système selon la revendication 1, dans lequel le dispositif d'annotation (154) est configuré pour générer automatiquement des annotations attribuées à des caractéristiques physiques dans une vue.

6. Système selon la revendication 5, dans lequel les annotations générées automatiquement peuvent inclure des caractéristiques et/ou des dispositifs d'intervention dans la vue.

7. Système selon la revendication 5, dans lequel les annotations (202) comprennent des formes géométriques, des contours, des mesures et des positions marquées.

8. Système selon la revendication 2, dans lequel le système de commande du guidage robotique (156) suit le mouvement des annotations pour ajuster la position de la sonde.

9. Système selon la revendication 1, comprenant en outre une interface (130) pour ajouter manuellement des annotations par l'intermédiaire du dispositif d'annotation.

10. Système selon la revendication 1, comprenant en outre un système d'avertissement (160) couplé au dispositif d'annotation pour surveiller les différences entre les annotations dans une vue actuelle et les annotations dans une vue précédente.

11. Système selon la revendication 1, comprenant en outre une interface (130) pour guider manuellement le robot à l'aide de la rétroaction sensorielle et des annotations comme référence.

12. Système d'imagerie, comprenant :
une première modalité d'imagerie (136) comprenant un système à rayons X et une seconde modalité d'imagerie (138) comprenant un système à ultrasons comportant une sonde d'échocardiographie transœsophagienne (TEE) ;
un dispositif d'annotation (154) configuré pour générer des annotations des cibles anatomiques (202, 204, 206, 208, 210) dans des images de la première modalité d'imagerie et/ou de la seconde modalité d'imagerie, chaque annotation comprenant un descripteur géométrique de la cible anatomique,
un module d'enregistrement (115) configuré pour fusionner les images des première et seconde modalités d'imagerie comprenant les annotations ;
un système de commande du guidage robotique (156) configuré pour guider un robot en fonction des annotations et d'une position mesurée du robot pour positionner et maintenir la sonde pour permettre de maintenir les positions et les orientations de vue attribuées ; et
au moins un dispositif d'affichage comportant un écran pour afficher une image fusionnée des première et seconde modalités d'imagerie de telle sorte que l'image fusionnée conserve une position et une orientation de vue attribuées.

13. Système selon la revendication 10, dans lequel la vue attribuée comprend une vue « en face » ou une vue mi-œsophagienne 4-chambre ou une vue selon l'axe longitudinal ou une vue transgastrique ou une vue aortique 3-feuillets valvulaires ou une vue dans le plan x.

14. Système selon la revendication 12, dans lequel le dispositif d'annotation (154) est configuré pour générer automatiquement des annotations attribuées à des caractéristiques physiques dans une vue.

15. Système selon la revendication 12, dans lequel les annotations (202) comprennent des formes géométriques, des contours, des mesures et des positions marquées.
